# EUROPEAN PATENT APPLICATION

(11) **EP 4 092 100 A1**
(43) Date of publication of application: **23.11.2022**
(21) Application number: 21174301.8
(22) Date of filing: 18.05.2021
(51) Int. Cl.: C12M 1/00, C12M 1/08

(54) **REACTOR AND METHODS FOR OPERATING SAID REACTOR**

(71) Applicant: Autark Energy Systems B.V., 7861 TB Oosterhesselen (NL)
(72) Inventor: JAGER, Filips Gustaaf Hendrik, 7861 TB OOSTERHESSELEN (NL)
(74) Representative: van Dam, Vincent

(57) **Abstract**

The present invention relates to a gas-lift reactor. The present invention also relates to a method for operating said gas-lift reactor and a method for growing organisms capable of photosynthesis in an aqueous liquid in a gas-lift photobioreactor.

## Description

The present invention relates to a gas-lift reactor. The present invention also relates to a method for operating said gas-lift reactor and a method for growing organisms capable of photosynthesis in an aqueous liquid in a gas-lift photobioreactor.

Reactors of the gas-lift type are well recognized as efficient contactors for processes involving gas, liquid and solid phases. Reactors of this type are typically designed as an inner chamber, such as a column, placed within an outer chamber, such as a tank. Gas distributors are placed on the bottom of the reactor to provide bubbles in the liquid medium contained in the reactor. The bubbling gas, typically air, but also other gases will be suitable, causes a local decrease in the density of the medium, causing an upward flow through the chamber in which the bubbles are released. As soon as the bubbles have reached the upper surface of the medium in the reactor the gas contained in the bubbles will be released in the air causing the density of the medium to increase. As a result the denser medium will enter the other chamber which is not provided at its bottom with a bubble generating means and the denser medium will flow downwards through that chamber. The chambers are in fluid connection at the bottom so that the down flowing medium that has reached the bottom will enter the chamber provided with said bubble generating means so that the medium is forced to flow up again. This way a circular flow is created.

The bubbles provide turbulence in the medium which is beneficial for mixing the contents of the reactor and which is in general sufficient in smaller type reactors. However the turbulence and mixing predominantly occurs in the range of the bubble track, in other words in vertical direction. This causes mixing limitations in case of larger reactors with multiple inner chambers, in particular because the contents of the reactor are insufficiently mixed outside the range of the vertically ascending bubble track. The present invention aims to overcome these limitations.

### Summary of the invention

In a first aspect the invention relates to a gas-lift reactor, comprising an outer chamber having a bottom portion and a top portion, one or more inner chamber having a bottom portion and a top portion, and configured within said outer chamber; wherein a longitudinal direction of the reactor is defined from top to bottom and a transversal plane being defined as the plane perpendicular to the longitudinal direction; wherein the bottom portion of the one or more inner chamber comprises a first area with one or more openings configured to allow fluid connection between the one or more inner chamber and said outer chamber on one first side of the one or more inner chamber; wherein the top portion of the one or more inner chamber comprises a second area with one or more openings configured to allow fluid connection between the one or more inner chamber and said outer chamber on one second side of the one or more inner chamber; and at least one bubble generating means configured to inject gas bubbles in the bottom portion of the outer chamber or in the bottom portion of the one or more inner chamber such to allow fluid circulation between the one or more inner chamber and outer chamber via said openings; wherein in the transversal plane the first and second area with one or more openings of each one or more inner chamber are offset with respect to one another.

In a second aspect the invention relates to a method wherein the reactor contains a liquid medium and wherein the method comprises providing a circulation between said outer chamber and inner chamber(s) by bubbling gas from said at least one bubble generating means, such that the direction of flow in said inner chambers is inverse to the direction of the flow in said outer chamber.

Because the opening(s) in the top portion of the inner chamber(s) are offset with respect to the opening(s) in the bottom area a liquid medium flowing from top to bottom in the inner chamber or vice versa will be transported not only in the longitudinal direction of the inner chamber, but also in a radial direction with respect to the longitudinal direction. Thus, in accordance with the invention the bottom portion of an inner chamber comprises one or more openings configured to allow a fluid stream between the inner chamber and said outer chamber in a first principle radial direction with respect to the longitudinal direction of the reactor. On the other hand the top portion of an inner chamber comprises one or more openings configured to allow a fluid stream between the inner chamber and said outer chamber in a second principle radial direction with respect to the longitudinal direction of the reactor. When the first principle radial direction is into the inner chamber, the second principle radial direction is out of the inner chamber and vice versa.

This results in improved mixing of the contents of the reactor because the liquid medium is forced to flow in an inner chamber from an entry opening to an exit opening in the longitudinal direction of the reactor and because the point of entry is offset in the transversal plane of the reactor with respect to the point of exit, the direction of flow also has a radial component, which may lead to a swirl of the contents of the inner chamber, thereby improving mixing of the contents of the reactor. On the other hand, because the point of entry of an inner chamber is offset in the transversal plane of the reactor with respect to the point of exit of that inner chamber, the direction of flow in the outer chamber will also have a radial component because the flow in the outer chamber will either be from a point of exit of an inner chamber to a point of entry of the same or another inner chamber depending on the design of the reactor. Either way, the provision of the reactor according to the invention allows for improved mixing of the contents in both the outer chamber and the inner chamber(s) by providing a passive means to control the flow of the liquid medium in the reactor, regardless whether the direction of flow of the liquid medium is from top to bottom or from bottom to top in the inner chamber(s).

The gas-lift reactor according to the invention can be applied in chemical engineering, metallurgy, fermentation processes, cultivating microorganisms or waste water treatment. This includes without limitation fermenters, sugar reactors and bioreactors. In a preferred embodiment the reactor is a bioreactor. Such a bioreactor may be designed for both heterotrophic and autotrophic organisms. Preferably the gas-lift reactor a photobioreactor, such as for cultivation of algae.

Therefore, in a third aspect the invention relates to a method for growing organisms capable of photosynthesis in an aqueous liquid in the reactor according to the invention, comprising providing a circulation between said outer chamber and inner chamber(s) by bubbling gas from said at least one bubble generating means, such that the direction of flow in said inner chamber(s) is inverse to the direction of the flow in said outer chamber, and exposing the organisms in the flowing aqueous liquid to light.

### Short description of the drawings

Fig.1 shows a schematic perspective view of a configuration of two inner chambers and a sparger within an outer chamber.
Fig.2A shows a schematic perspective view of a gas-lift reactor according to the invention with multiple inner chambers; Fig.2b shows a top view of the reactor of Fig. 2A.
Fig.3 shows a schematic top view of a configuration of multiple inner chambers arranged around light bars in an air-lift photobioreactor according to the invention.

### Detailed description of the invention

The gas-lift reactor, and in particular according to the invention comprises an outer chamber having a bottom portion and a top portion, one or more inner chamber having a bottom portion and a top portion, and configured within said outer chamber. The outer chamber may suitably be configured as a tank or a basin filled with a liquid medium when in operation and wherein the inner chambers are submerged in the liquid medium at least with their entry and exit openings, i.e. the first and second area with the one or more openings. This way the submerged openings allow fluid connection between said at least one inner chamber and said outer chamber via the bottom and the top portion of the inner chamber. Because the inner chamber is placed in the outer chamber, the wall of an inner chamber preferably separates the outer and inner chamber from each other. In a suitable embodiment the outer chamber is defined by a first vertical cylinder and the at least one inner chamber is defined by a second vertical cylinder, wherein at least one inner chamber is mounted into said first vertical cylinder as a further "second cylinder".

The bottom portion of an inner chamber comprises a first area with one or more openings configured to allow fluid connection between this inner chamber and said outer chamber on one first side of this inner chamber, whereas the top portion of the inner chamber comprises a second area with one or more openings configured to allow fluid connection between the inner chamber and said outer chamber on one second side of the inner chamber.

If the first area with one or more openings in the bottom portion of an inner chamber functions as an entry for a liquid medium into the inner chamber when the reactor is in operation, the second area with one or more openings in the top portion of the inner chamber functions as an exit for the liquid medium out of the inner chamber.

On the other hand, if the first area with one or more openings in the bottom portion of an inner chamber functions as an exit for a liquid medium out of the inner chamber when the reactor is in operation, the second area with one or more openings in the top portion of the inner chamber functions as an entry for the liquid medium into the inner chamber.

The first and second areas with one or more openings for exit and entry of medium are each arranged on one lateral side of the inner chambers. In other words, liquid medium can only enter the inner chamber on a single lateral side and liquid medium can only exit the inner chamber on a single lateral side. In accordance with the invention, the inner chambers therefore do not contain points of entry and exit for flowing liquid medium on the top and bottom ends of the inner chambers. Thus, if the inner chambers are configured as cylinders, the bottom and top ends of the cylinders do not function as points of entry or exit for the circulating liquid medium, and in view of this the bottom end is closed and the top end of such a cylinder is either not submerged in the medium or closed.

In an embodiment wherein the reactor is configured to have a flow of liquid medium from top to bottom in the inner chambers/cylinders, the bottom end is closed and the top end of such an inner chamber/cylinder is either not submerged in the medium or, preferably, closed. On the other hand, in an embodiment wherein the reactor is configured to have a flow of liquid medium from bottom to top in the inner chambers/cylinders, it may be preferred that the inner chambers/cylinders contain a lateral exit for flowing liquid medium in the top portion and an open top which is not submerged in the liquid medium so that gas bubbles rising in the inner chambers/cylinders can be efficiently released on the medium surface. This is opposed to prior art gas-lift reactor inner chambers which have multiple openings distributed over the full circumference of the inner chambers or which have an open end, such that entry and exit of flowing medium takes place over the full circumference of the inner chambers.

The first and second area with one or more openings with regard to the inner chamber are offset with respect to one another in the transversal plane. Because the opening(s) in the top portion of the inner chamber(s) are offset with respect to the opening(s) in the bottom area a liquid medium flowing from top to bottom in the inner chamber or vice versa will be transported not only in in the longitudinal direction of the inner chamber, but also in a radial direction with respect to the longitudinal direction.

Thus, in accordance with the invention the bottom portion of an inner chamber may comprise a lateral exit for a fluid stream in a first principle radial direction with respect to the longitudinal direction of the reactor, while the top portion of the inner chamber a lateral entry for a fluid stream in a second principle radial direction with respect to the longitudinal direction of the reactor. Alternatively, the bottom portion of an inner chamber comprises may comprise a lateral entry for a fluid stream in a first principle radial direction with respect to the longitudinal direction of the reactor, while the top portion of the inner chamber a lateral exit for a fluid stream in a second principle radial direction with respect to the longitudinal direction of the reactor. In other words, when the first principle radial direction is into the inner chamber, the second principle radial direction is out of the inner chamber and vice versa. The first and second principle direction may be fully in line with the transversal (horizontal) plane but may also be under a small angle upwards or downwards with respect to the horizontal plane, this should be a sharp angle, preferably less than 45°, such as less than 40°, less than 30°, less than 20°, less than 10°, less than 5°, most preferably the first and second principle direction are in horizontal direction.

In principle any degree of offset in the transversal plane of the lateral entry with respect to the lateral exit of an inner chamber will be advantageous for mixing the reactor components to a certain extent. In this respect a first area with one or more openings in the bottom area of an inner chamber may be under an acute (<90°), right (90°) or obtuse angle (>90° and < 180°), as seen in the transversal plane, with respect to a second area with one or more openings in the top portion of an inner chamber. A first area with one or more openings in the bottom area of an inner chamber may also be arranged opposite as seen in the transversal plane, i.e. under a degree of 180° with respect to a second area with one or more openings in the top portion of an inner chamber.

It is preferred that a first area with one or more openings in the bottom area of an inner chamber is under an obtuse angle (>90°), as seen in the transversal plane, with respect to a second area with one or more openings in the top portion of an inner chamber, for instance 120°, because this forces the flowing liquid medium in a vertical direction, as well as a radial direction in two dimensions of the transversal plane with maximal lateral distance between entry and exit. This leads to optimal mixing of the contents of the inner chamber.

In a preferred embodiment the reactor according to the invention comprises multiple inner chambers. In this embodiment the gas-lift reactor comprises multiple inner chambers, having a bottom portion and a top portion, and configured within said outer chamber; wherein the bottom portion of each inner chamber comprises a first area with one or more openings configured to allow fluid connection between an inner chamber and said outer chamber on one first side of each inner chamber; wherein the top portion of each inner chamber comprises a second area with one or more openings configured to allow fluid connection between each inner chamber and said outer chamber on one second side of each inner chamber; and at least one bubble generating means configured to inject gas bubbles in the bottom portion of the outer chamber or in the bottom portion of each inner chamber such to allow fluid circulation between the inner chambers and outer chamber via said openings; wherein in the transversal plane the first and second area with one or more openings with regard to each inner chamber are offset with respect to one another. The configuration of the inner chambers allows to incorporate a large number of inner chambers in an outer chamber and therefore allows upscaling of the gas-lift reactors. The offset configuration of the entry and exit of the inner chambers in a multi inner chamber embodiment allows for further optimisation of mixing in the outer chamber because inner chambers can be arranged such with respect to each other that liquid medium flowing out of an exit of a first inner chamber can flow to an entry of a second inner chamber crossing the full area of the outer chamber between two inner chambers, which lead to a swirl of flowing liquid medium throughout the full reactor, which further improves contacting and mixing of the components within the liquid medium.

In one suitable embodiment of a multi inner chamber gas-lift reactor in accordance with the invention the bottom portion of a first inner chamber comprises a first area with one or more openings on one side of the first inner chamber; wherein the top portion of a second inner chamber adjacent to said first inner chamber comprises a second area with one or more openings on one side of the second inner chamber; wherein in the transversal plane said first area with one or more openings of the first inner chamber is offset with respect to said second area with one or more openings of the second inner chamber. In other words the exit of a first inner chamber is offset, as seen in the transversal plane, with respect to the entry of a second chamber adjacent to the first inner chamber. This forces the flowing liquid medium in a vertical direction in the outer chamber, as well as a radial direction in two dimensions of the transversal plane with maximal lateral distance between exit out of the first inner chamber and entry into the second inner chamber. This leads to optimal mixing of the contents of the outer chamber.

In another suitable embodiment of a multi inner chamber gas-lift reactor in accordance with the invention the bottom portion of a first inner chamber comprises a first area with one or more openings on one side of the first inner chamber; wherein the top portion of a second inner chamber adjacent to said first inner chamber comprises a second area with one or more openings on one side of the second inner chamber; wherein in the transversal plane said first area with one or more openings of the first inner chamber is aligned with said second area with one or more openings of the second inner chamber. In other words the exit of a first inner chamber is aligned, as seen in the transversal plane, with the entry of a second chamber adjacent to the first inner chamber. Although this setup forces the flowing liquid medium in a shorter flow path between exit out of the first inner chamber and entry into the second inner chamber than the previous embodiment, this setup may be preferred in gas-lift reactors multiple inner chambers arranged around other reactor components, such as vertical reactor components, such a light bars. This allows a practical design of the inner chambers with respect to each other and ensures uniform flow conditions throughout the outer chamber.

The vertical reactor components discussed above may suitably be in the form of light bars, such as LED bars, in particularly when the reactor is a photobioreactor. In this case the outer chamber may comprise a plurality of light bars, the individual light bars extending from the top portion of the outer chamber in the direction of the bottom portion of the outer chamber. The downward, in particular vertical, arrangement of the light bars allows the use of multiple inner chambers in a single outer chamber and allows to cultivate large volumes of organisms, such as algae, in the reactor.

As mentioned above, in case of vertical components other than inner chambers in the reactor it is preferred the exit of a first inner chamber is aligned, as seen in the transversal plane, with the entry of a second chamber adjacent to the first inner chamber. In a preferred setup of a photobioreactor therefore inner chambers are arranged around each light bar in a hexagonal pattern, wherein within the hexagon the bottom portion of each inner chamber comprises an area with one or more openings aligned in the transversal plane with respect to an area with one or more openings in the top portion of an inner chamber adjacent to the other inner chamber, wherein the area with one or more openings in the bottom area of each inner chamber is under an angle of 120° as seen in the transversal plane, with respect to the area of with one or more openings in the top portion of the same inner chamber.

In other words, each inner chamber has an entry which, as seen in the transversal plane, is under an angle of 120° with the exit, while the exit of one inner chamber is aligned, i.e. under an angle of 180°, with the entry of one another, adjacent chamber. This setup allows to connect multiple inner chambers to create optimal mixing and light distribution throughout the flowing and growing organisms. In view of the above, the present invention also preferably relates to a method for growing organisms capable of photosynthesis in an aqueous liquid in the photobioreactor described above, providing a circulation between said outer chamber and inner chambers by bubbling gas from said at least one bubble generating means, such that the direction of flow in said inner chambers is inverse to the direction of the flow in said outer chamber, thereby exposing the organisms in the flowing aqueous liquid to said light bars in said outer chamber.

In order to function as a gas-lift reactor, the reactor according to the invention comprises at least one bubble generating means, such as a sparger, configured to inject gas bubbles in the bottom portion of the outer chamber or in the bottom portion of the inner chamber such to allow fluid circulation between the inner chamber and outer chamber via said openings. The bubbling gas causes a local decrease in the density of the growth medium, causing upward flow through chamber in which the bubble generating means is positioned. As soon as the bubbles have reached the upper surface the gas contained in the bubbles will be released causing the density of the medium to increase. As a result the denser medium will enter the other chamber which is not provided at its bottom with a bubble generating means and the denser medium will flow downwards. Once at the bottom the dense medium exits the chamber to enter the chamber in which the bubble generating means is provided and the cycle is repeated so that a circular flow is obtained.

In a preferred embodiment the reactor comprises at least one bubble generating means located in the outer chamber in the bottom portion of the outer chamber and at a lateral distance from the bottom end of the or each inner chamber. The lateral distance is chosen such that bubbles are injected in the outer chamber and cannot accidentally enter into the inner chamber. This allows unhindered circulation of the liquid growth medium. In this embodiment the bubbling gas causes a local decrease in the density of the growth medium, causing upward flow through the outer chamber. As soon as the bubbles have reached the upper surface the gas contained in the bubbles will be released causing the density of the medium to increase. As a result the denser medium will enter the at least one inner chamber which is not provided at its bottom with a bubble generating means and the denser medium will flow downwards through the at least one inner chamber. The bottom of the at least one inner chamber is in fluid contact with the medium in the outer chamber so that the down flowing medium that has reached the bottom of the at least one inner chamber will be caused to enter the outer chamber which is provided with said bubble generating means so that the medium is forced to flow up again. Thus, a circulation between said outer chamber and one or more inner chambers is provided by bubbling gas from said at least one bubble generating means, such that the direction of flow in said outer chamber is upwards, and the direction of flow in said inner chamber(s) is downwards. This way a circular flow is created. This configuration is preferred for the following reasons. The volume of the inner chamber(s) will always be smaller than the volume of the outer chamber. As mentioned in the introduction the bubbles provide turbulence in the medium which is beneficial for mixing the contents of the reactor. The radial flow through the outer chamber as a result of the offset arrangement of the entry and exit of the inner chamber further contributes to that. Thus, by the provision of the bubble generating means in the outer chamber a larger volume of medium is subjected to optimal mixing conditions. It is preferred in this respect that in a multi inner chamber setup, the at least one bubble generating means is configured to generate bubbles in a central area in the transversal plane between a first area with one or more openings in the bottom portion of a first inner chamber and a second area with one or more openings in the top portion of a second inner chamber adjacent to the first inner chamber. The provision of the bubble generating means in a central area of the bottom of the reactor between one inner chamber and an adjacent inner chamber increases the volume of medium flowing between two inner chambers and minimizes the amount of medium ascending vertically. The flow in radial direction can be controlled by adjusting the size of the entry and/or exit openings. In order to further optimize flow between two adjacent inner chambers in case of upward flow in the outer chamber and descending flow in the inner chamber(s), it is preferred that the total size (that is the diameter size) of the one or more openings in the bottom portion of the inner chamber(s) is smaller than the total size of the one or more openings in the top portion of the inner chamber(s). This way the speed of flow into the outer chamber is increased, which further contributes to improved mixing of the contents of the reactor, in particular in view of the larger volume of the outer chamber compared to the volume of the inner chambers, as explained above.

The speed of flow into the outer chamber or inner chamber(s) can further be regulated by increasing the amount of bubbles generated in a period of time. A higher rate of bubbling causes a higher speed of upward flow, which on its turns results also in a higher downward flow speed, but also a higher radial flow speed. A higher bubbling rate therefore further increases mixing of the reactor contents in all directions.

Although not of particular preference, it is also possible that the inner chamber(s) function as riser chambers, because the principles of the present invention also fully apply to this configuration. In this case the reactor according to the invention comprises at least one bubble generating means located in the bottom portion of the or each inner chamber, instead of in the outer chamber. In this case a circulation between said outer chamber and one or more inner chamber is provided by bubbling gas from said at least one bubble generating means in the inner chamber(s), such that the direction of flow in said outer chamber is downwards, and the direction of flow in said one or more inner chamber is upwards.

### Detailed description of the drawings

The invention will now be further elucidated in the attached drawings. The following explanation is meant to illustrate and explain the invention and not to limit the claims.

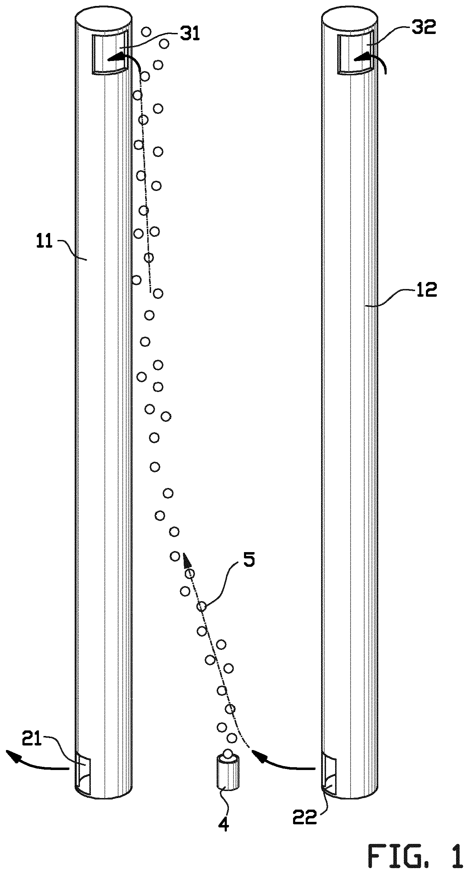

The principle of the invention can be explained by means of Fig.1, which shows a schematic perspective view of a configuration of two inner chambers in the form of cylinders 11 and 12 and a sparger 4 within an outer chamber (not shown). Sparger 4 produces bubbles 5. The bubbles 5 allow a circulation between said outer chamber and inner chamber(s), such that a liquid medium flows from top to bottom in cylinders 11 and 12 and from bottom to top in the outer chamber The liquid medium may enter the cylinders 11 and 12 via entry openings 31 and 33 and exit the cylinders 11 and 12 via exit openings 21 and 22 (see arrows for the principle direction of flow of liquid medium). Liquid medium exiting cylinder 12 via exit opening 22 exits cylinder 12 in a substantially horizontal direction towards sparger 4. Because the openings 31, 32 in the top portion of the inner chamber are offset with respect to the opening 21, 22 in the bottom area a liquid medium flowing from top to bottom in each inner chamber 11, 12 will be transported not only in in the longitudinal direction (top to bottom) of each inner chambers 11, 12, but also in a radial direction with respect to the longitudinal direction. This improves mixing in the inner chambers. On the other hand, because the point of entry 31, 32 of an inner chamber is offset in the transversal plane with respect to the point of exit 21, 22 of that inner chamber, the direction of flow in the outer chamber will also have a radial component because the flow in the outer chamber will be from point of exit 22 of inner chamber 12 to point of entry 31 of inner chamber 11. In Fig.1 the exit 22 of inner chamber 12 is offset, as seen in the transversal plane, with respect to the entry 31 of inner chamber 11. This forces the flowing liquid medium in a vertical direction in the outer chamber, as well as a radial direction in two dimensions of the transversal plane with maximal lateral distance between exit out inner chamber 12 and entry into inner chamber 11. This leads to optimal mixing of the contents of the outer chamber. The diameter size of the openings 21, 22 in the bottom portion of the inner chambers is smaller than the diameter size of the openings 31, 32 in the top portion of the inner chambers. This way the speed of flow into the outer chamber is increased, which further contributes to improved mixing of the contents of the reactor.

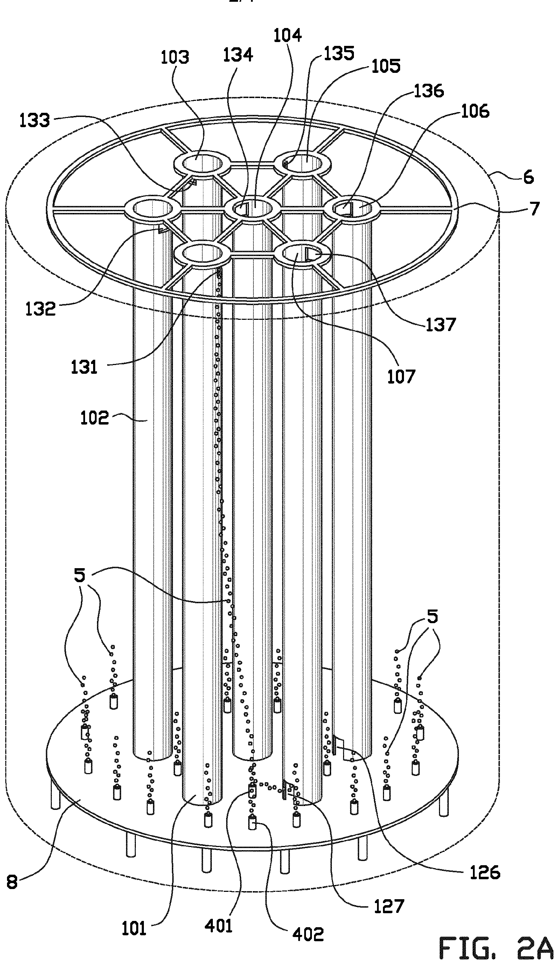

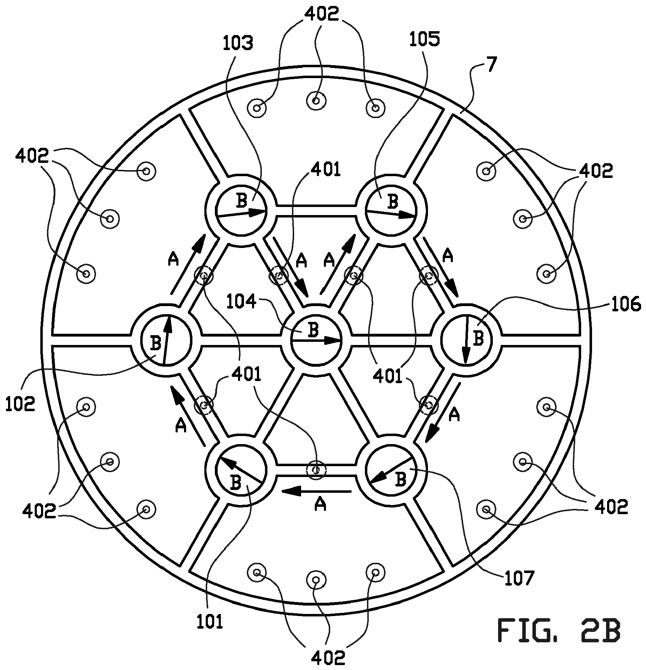

Fig.2A shows a schematic perspective view of a gas-lift reactor according to the invention with multiple inner chambers 101, 102, 103, 104, 105, 106, 107 in the form of cylinders distributed in outer chamber 6 and mounted onto supporting plate 8 and supporting frame 7; Fig.2b shows a top view of the reactor of Fig. 2A. The bottom portion of each inner chamber 101, 102, 103, 104, 105, 106, 107 comprises a first lateral opening, of which openings 126 and 127 of inner chambers 106 and 107, respectively are visible in Fig.2A. These lateral openings in the bottom portion are configured to allow flow of a liquid medium out of the inner chambers 101, 102, 103, 104, 105, 106, 107 into the outer chamber 6. The bottom end of each inner chamber is closed by supporting plate 8. The top portion of each inner chamber 101, 102, 103, 104, 105, 106, 107 comprises a second lateral opening 131, 132, 133, 134, 135, 136, 137 configured to allow flow of liquid medium from said outer chamber 6 into the inner chambers 101, 102, 103, 104, 105, 106, 107. When in operation the top ends of the inner chambers are closed, for instance with a sealing lid (not shown for the sake of visualisation of the openings in the top portions. Spargers 401 are configured to generate bubbles in a central area in the transversal plane between inner chambers 101 and 102, inner chambers 102 and 103, inner chambers 103 and 104, inner chambers 104 and 105, inner chambers 105 and 106, inner chambers 106 and 107 and between inner chambers 107 and 101. Spargers 402 are also provided on the inner circumference of the outer chamber 6 so as to avoid caking of components to the wall of the outer chamber. The provision of the spargers in the bottom portion of the outer chamber and at a lateral distance from the bottom end each inner chamber results a circulation between said outer chamber 6 and inner chambers 101, 102, 103, 104, 105, 106, 107 by bubbling gas from said spargers 401, such that the direction of flow in said outer chamber 6 is upwards, and the direction of flow in said inner chambers 101, 102, 103, 104, 105, 106, 107 is downwards. Because the openings 131, 132, 133, 134, 135, 136, 137 in the top portion of the inner chamber are offset with respect to the openings 121, 122, 123, 124, 125 (openings 121-125 are not visible in Fig. 2) and 126, 127 in the respective bottom areas a liquid medium flowing from top to bottom in each inner chamber 101, 102, 103, 104, 105, 106, 107 will be transported not only in in the longitudinal direction (top to bottom) of each inner chambers 11, 12, but also in a radial direction with respect to the longitudinal direction, of which the vector is indicated with arrow B. This improves mixing in the inner chambers. On the other hand, because the point of entry of each inner chamber is offset in the transversal plane with respect to the point of exit of that inner chamber, the direction of flow in the outer chamber will also have a radial component of which the vector is indicated with arrow A because the flow in the outer chamber will also be from point of exit 121 of inner chamber 101 to point of entry 132 of inner chamber 102, from point of exit 122 of inner chamber 102 to point of entry 133 of inner chamber 103, from point of exit 123 of inner chamber 103 to point of entry 134 of inner chamber 104, from point of exit 124 of inner chamber 104 to point of entry 135 of inner chamber 105, from point of exit 125 of inner chamber 105 to point of entry 136 of inner chamber 106, from point of exit 126 of inner chamber 106 to point of entry 137 of inner chamber 107 and from point of exit 127 of inner chamber 107 to point of entry 131 of inner chamber 101. While the vectors are indicated with straight arrows A and B, these vectors are in effect the result of a swirling movement of flowing liquid medium. This way a swirl of flowing liquid medium is obtained throughout the full reactor, which is advantageous for contacting and mixing of the components within the liquid medium.

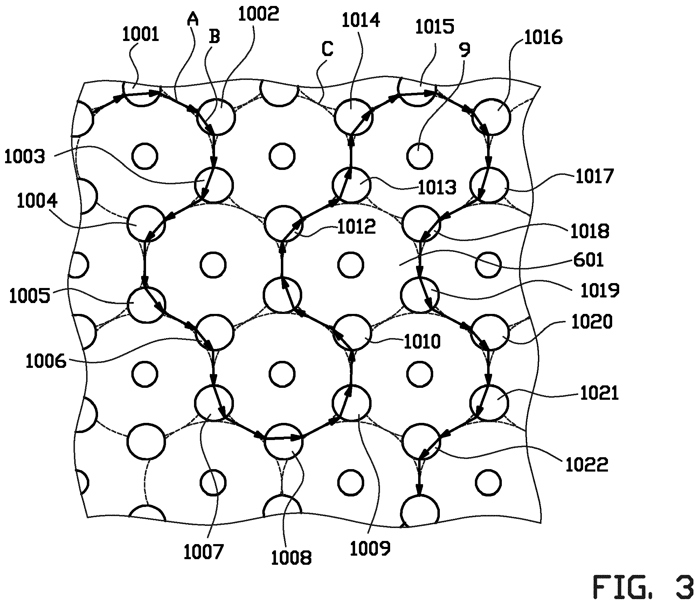

Fig. 3 shows a schematic top view of a configuration of multiple inner chambers 1001, 1002, 1003, 1004, 1005, 1006, 1007, 1008, 1009, 1010, 1011, 1012, 1013, 1014, 1015, 1016, 1017, 1018, 1019, 1020, 1021, 1022 arranged around light bars 9 in an air-lift photobioreactor according to the invention. The light bars 9 emit light visualized by the dotted line circles C. Spargers (not shown) provided in the outer chamber 601 blow bubbles so that the direction of flow in said outer chamber 601 is upwards, and the direction of flow in said inner chambers 1001 - 1022 is downwards. In this photobioreactor inner chambers 1001 - 1022 are arranged around each light bar 9 in a hexagonal pattern wherein the bottom portion of a first inner chamber comprises an lateral exit aligned in the transversal plane, i.e. as seen from above, with respect to entry in the top portion of a second inner chamber adjacent to the first inner chamber, wherein the lateral exit in the bottom area of each inner chamber is under angle of 120° as seen from above, with respect to the lateral entry in the top portion of the same inner chamber. In other words, each inner chamber has an entry which, as seen from above, is under an angle of 120° with the exit, while the exit of one inner chamber is aligned, i.e. under an angle of 180°, with the entry of one another, adjacent chamber. In the present case the exit of inner chamber 1001 is aligned, i.e. under an angle of 180°, with the entry of adjacent chamber 1002, the exit of inner chamber 1002 is aligned, i.e. under an angle of 180°, with the entry of adjacent chamber 1003, and so on. This setup allows to connect high number of multiple inner chambers as visualized with vector arrows A and B to create optimal mixing in both the inner chambers and the outer chamber with optimal light distribution throughout the flowing and growing organisms. As explained under Fig. 2, while the vectors are indicated with straight arrows A and B, these vectors are in effect the result of a swirling movement of flowing liquid medium.

## Claims

1. Gas-lift reactor, comprising
an outer chamber having a bottom portion and a top portion, one or more inner chamber having a bottom portion and a top portion, and configured within said outer chamber;
wherein a longitudinal direction of the reactor is defined from top to bottom and a transversal plane being defined as the plane perpendicular to the longitudinal direction;
wherein the bottom portion of the one or more inner chamber comprises a first area with one or more openings configured to allow fluid connection between the one or more inner chamber and said outer chamber on one first side of the one or more inner chamber;
wherein the top portion of the one or more inner chamber comprises a second area with one or more openings configured to allow fluid connection between the one or more inner chamber and said outer chamber on one second side of the one or more inner chamber; and
at least one bubble generating means configured to inject gas bubbles in the bottom portion of the outer chamber or in the bottom portion of the one or more inner chamber such to allow fluid circulation between the one or more inner chamber and outer chamber via said openings;
wherein in the transversal plane the first and second area with one or more openings of each one or more inner chamber are offset with respect to one another.

2. Gas-lift reactor according to claim 1, comprising multiple inner chambers, having a bottom portion and a top portion, and configured within said outer chamber;
wherein the bottom portion of each inner chamber comprises a first area with one or more openings configured to allow fluid connection between an inner chamber and said outer chamber on one first side of each inner chamber;
wherein the top portion of each inner chamber comprises a second area with one or more openings configured to allow fluid connection between each inner chamber and said outer chamber on one second side of each inner chamber; and
at least one bubble generating means configured to inject gas bubbles in the bottom portion of the outer chamber or in the bottom portion of each inner chamber such to allow fluid circulation between the inner chambers and outer chamber via said openings;
wherein in the transversal plane the first and second area with one or more openings with regard to each inner chamber are offset with respect to one another.

3. Reactor according to claim 2, wherein the bottom portion of a first inner chamber comprises a first area with one or more openings on one side of the first inner chamber;
wherein the top portion of a second inner chamber adjacent to said first inner chamber comprises a second area with one or more openings on one side of the second inner chamber;
wherein in the transversal plane said first area with one or more openings of the first inner chamber is offset with respect to said second area with one or more openings of the second inner chamber.

4. Reactor according to claim 2, wherein the bottom portion of a first inner chamber comprises a first area with one or more openings on one side of the first inner chamber;
wherein the top portion of a second inner chamber adjacent to said first inner chamber comprises a second area with one or more openings on one side of the second inner chamber;
wherein in the transversal plane said first area with one or more openings of the first inner chamber is aligned with said second area with one or more openings of the second inner chamber.

5. Reactor according to any of the previous claims, wherein the outer chamber comprises a plurality of light bars, the individual light bars extending from the top portion of the outer chamber in the direction of the bottom portion of the outer chamber.

6. Reactor according to claim 5, wherein around each light bar inner chambers are arranged in a hexagonal pattern and wherein within the hexagon the bottom portion of each inner chamber comprises a first area with one or more openings aligned in the transversal plane with respect to a second area with one or more openings in the top portion of an inner chamber adjacent to the other inner chamber, wherein the first area with one or more openings in the bottom area of each inner chamber is under an angle of 120° as seen in the transversal plane, with respect to a second area of with one or more openings in the top portion of the same inner chamber.

7. Reactor according to any of the previous claims, comprising at least one bubble generating means located in the outer chamber in the bottom portion of the outer chamber and at a lateral distance from the bottom end of the or each inner chamber.

8. Reactor according to claim 7, wherein at least one bubble generating means is configured to generate bubbles in a central area in the transversal plane between a first area with one or more openings in the bottom portion of a first inner chamber and a second area with one or more openings in the top portion of a second inner chamber adjacent to the first inner chamber.

9. Reactor according to claim 7 or 8, wherein the total size of the one or more openings in the bottom portion of the inner chambers is smaller than the total size of the one or more openings in the top portion.

10. Reactor according to any of the claims 1 to 6, comprising at least one bubble generating means located in the bottom portion of the or each inner chamber.

11. A method for operating a gas-lift reactor according to any of the previous claims,
wherein the reactor contains a liquid medium and wherein the method comprises providing a circulation between said outer chamber and one or more inner chambers by bubbling gas from said at least one bubble generating means, such that the direction of flow in said one or more inner chamber is inverse to the direction of the flow in said outer chamber.

12. Method according to claim 11, wherein the reactor is the reactor according to any of the claims 1 to 9, and comprising providing a circulation between said outer chamber and one or more inner chambers by bubbling gas from said at least one bubble generating means, such that
the direction of flow in said outer chamber is upwards, and
the direction of flow in said one or more inner chamber is downwards.

13. Method according to claim 11, wherein the reactor is the reactor according to any of the claims 1 to 6 and 10,
and comprising providing a circulation between said outer chamber and one or more inner chamber by bubbling gas from said at least one bubble generating means, such that
the direction of flow in said outer chamber is downwards, and
the direction of flow in said one or more inner chamber is upwards.

14. Method according to claim 11, 12 or 13, wherein the method is directed to chemical engineering, metallurgy, fermentation processes, cultivating microorganisms or waste water treatment.

15. A method for growing organisms capable of photosynthesis in an aqueous liquid in the reactor according to any of the claims 1 to 10 comprising providing a circulation between said outer chamber and inner chamber(s) by bubbling gas from said at least one bubble generating means, such that the direction of flow in said inner chamber(s) is inverse to the direction of the flow in said outer chamber, and exposing the organisms in the flowing aqueous liquid to light.
